Europäisches Patentamt

(19) European Patent Office

Office européen des brevets

(11) Publication number: **0 009 911**
**B1**

(12) **EUROPEAN PATENT SPECIFICATION**

(45) Date of publication of patent specification: **08.06.83**

(51) Int. Cl.³: **A 61 M 5/14, F 16 K 7/00**

(21) Application number: **79301931.6**

(22) Date of filing: **18.09.79**

(54) **Flush valve.**

(30) Priority: **22.09.78 US 944751**

(43) Date of publication of application:
**16.04.80 Bulletin 80/8**

(45) Publication of the grant of the patent:
**08.06.83 Bulletin 83/23**

(84) Designated Contracting States:
**AT BE CH DE FR GB IT LU NL SE**

(56) References cited:
**EP - A - 0 006 761**
**DE - C - 628 167**
**DE - C - 897 930**
**DE - U - 1 970 313**
**US - A - 2 946 555**

(73) Proprietor: **NORTON COMPANY**
**1 New Bond Street**
**Worcester Massachusetts 01606 (US)**

(72) Inventor: **Hicks, Roger D.**
**4785 Mayfair Road**
**North Canton Ohio (US)**

(74) Representative: **Baillie, Iain Cameron et al,**
**c/o Ladas & Parry Isartorplatz 5**
**D-8000 München 2 (DE)**

Courier Press, Leamington Spa, England

Flush valve

The present invention is concerned with a flush valve for use with a catheter and is specifically directed to an improved valve for permitting the rapid flushing of an intravascular catheter used in a system for monitoring conditions in the thoracic cavity during surgery, in an intensive care ward, in cardio vascular diagnostic laboratories etc.

There have recently come on the market several devices for the continuous flushing of intravascular catheters wherein a glass capillary tube, such as a marine bore, is provided to limit the flushing to a very small amount. At the time of installing the catheter it is necessary to have a fast flush in order to completely fill the catheter with the flushing solution to prevent the danger of introducing an air bubble into the body of the patient whose condition is being monitored. One such catheter is made in accordance with the teachings of U.S. Patent 3,675,891. In that device a constant flowing capillary is provided in a separate chamber in the main body portion from that containing the flushing valve. The latter requires a pulling by a valve stem for the flushing to be initiated.

DE—U—1,970,313, dealing with valves for vehicles, discloses a valve which comprises a relatively rigid central portion and a flexible outer portion surrounding said central portion and having a sealing surface which engages said central portion around its whole circumference in liquid sealing relationship. An integral pair of lever arms molded as part of said outer portion extend from said portion radially of its axis for transmitting a shape-deforming force to said flexible outer portion to lift a portion of said sealing surface out of contact with the relatively rigid central portion to permit passage of liquid through said valve.

Other prior art dealing with valve devices is represented by U.S. Patents 2,706,101; 2,946,555; 3,985,140; 3,901,246 and 4,018,231. None of these patents shows an integral molded pair of lever arms. U.S. Patent 2,706,101 shows a valve comprising a rubber sleeve with an internal resilient flange disposed about a conduit wherein lateral compression of the sleeve causes distortion of the internal flange to permit flow of fluid between the sleeve and the conduit.

The earlier European patent application EP—A1—0,006,761, citable under EPC Art. 54(3) as regards its United States priority date of 29 June 1978, discloses a flow regulating device having the features of claim 1 of the present application but without radially extending lever arms.

In accordance with the present invention there is provided a flush valve for use with a catheter, which comprises a relatively rigid, elongated central portion, a main body including a flexible outer portion surrounding said central portion, said flexible outer portion having a sealing surface which engages said central portion around its whole circumference in liquid sealing relationship, an inlet passageway in said body adjacent one axial end of said central portion, an outlet passageway in said body adjacent the other axial end of said central portion, and an integral pair of lever arms molded as part of said body and extending radially of an axis parallel to that of the elongated central portion, said lever arms being adapted to transmit a shape-deforming force to said flexible outer portion to lift a portion of said sealing surface out of contact with the rigid central portion to permit passage of liquid from said inlet passageway to said outlet passageway, said central portion including an axially extending capillary continuously communicating said inlet passageway and said outlet passageway. Preferably the rigid central portion is cylindrical and the flexible outer portion contains a circular sealing ring and the deformation force converts the sealing surface from a circle to an oblong to lift a portion of the sealing surface from the central cylindrical portion. Preferably the main body includes two parallel tubular portions, one of said portions containing the axially extending central portion and the other tubular portion containing a separate passage for connection at one end to a pressure-sensing device and communicating at the other end with the outlet passageway, the portion of the main body comprising said two tubular portions constituting one lever arm, the other lever arm being solid and extending at an angle with respect to said main body portion containing said parallel tubular portions.

In the present invention, the radially extending lever arms permit simple one-hand manipulation, which would not be readily feasible with the device shown in U.S. Patent 3,675,891. The central elongated portion of the present invention contains the capillary tube, which is continuously open to permit continuous flow at a low rate in combination with intermittent rapid flushing controlled by the valve. The capillary tube is generally made of glass and is thus apt to be fragile. Therefore, the lever arms act about an axis which is parallel to the axis of the solid central portion.

In DE—U—1,970,313 there is no capillary tube present to permit continuous flow at a low rate. The lever arms shown in Figure 8 require activation by a separate pressure element.

Brief Description of the Drawings
Fig. 1 is a diagrammatic sectional view of the valve of the present invention.
Fig. 2 is a diagrammatic sectional view along the line 2—2 of Fig. 1 with the valve in the closed position.
Fig. 3 is a sectional view similar to Fig. 2 taken on line 3—3 of Fig. 1 with the valve in the flush position.

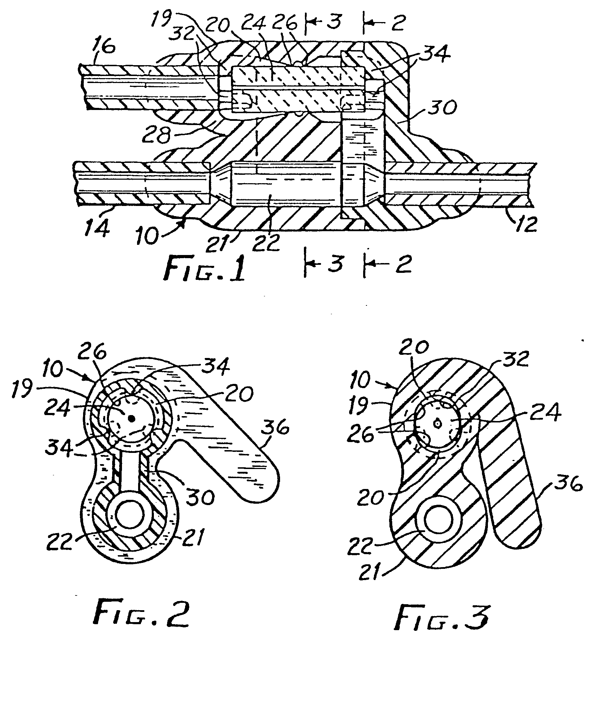

Referring to Figs. 1 and 2 the valve body 10 has three tubes 12, 14 and 16 connected thereto. Tube 12 is designed to be connected to the catheter, tube 14 is adapted to be connected to a pressure monitoring device and tube 16 is arranged to be connected to a pressurized source of flushing solution. The valve body 10 contains two tubular portions 19 and 21 containing passages 20 and 22 respectively. In passage 20 there is provided a glass "marine bore" capillary 24 having a cylindrical outer circumference. A flushing solution is prevented from by-passing the marine bore capillary 24 by cylindrical sealing ridges 26 formed in the interior portion of the body 10 defining the cavity 20.

The body 10 is preferably formed of a pliable plastic or elastomer such as urethane having a Shore A durameter of approximately 80. This body 10 is preferably formed of two pieces the major portion of which includes end piece 28 having two nipples which are integrally molded into the two tubes 14 and 16 respectively. A second end piece 30 having a single nipple is integrally molded into the tube 12.

The marine bore cylinder 24 is held in position centrally of the cavity 20 by means of shoulders 32 on end piece 28 which cooperate with shoulders 34 on cap 30. These shoulders 32 and 34 do not extend around the circumference of the cavity 20 so they do not interfere with the flow of the flushing fluid.

In assembling the device, marine bore tube 24 is inserted into the cavity 20 until it is held in position by the shoulders 32. The cap 30 is then inserted and sealed by use of an appropriate adhesive with the shoulders 34 engaging the other end of the marine bore capillary. As will be noted the two sealing lips 26 tightly engage the complete circumference of the marine bore capillary to make a tight seal therewith.

When the device is fully assembled and pressurized, liquid is fed from the tube 16 into cavity 20 and passes through the marine bore capillary 24 at a very low rate of flow, of the order of a few cm³ per hour.

Referring now to Figs. 2 and 3 there is shown a cross-sectional view of Fig. 1. In Fig. 2 the valve is in normal closed position with the sealing lips 26 in sealing engagement with the outside of the capillary tube 24. This figure also shows the integral lever arm 36 which is molded as a portion of the body 10 as can be seen in Fig. 2; the tubular portion 21 and the arm 36 form an integral pair of lever arms which are molded as an integral part of said body. These arms extend radially from tubular body 19 with respect to an axis parallel to that of the elongated central portion or capillary tube 24.

In Fig. 3 the lever arm 36 is shown as being moved towards the second lever arm (tube 21) so as to distort the body 19 of the valve adjacent passage 20. This distortion lifts a portion of the sealing rings 26 from the circumference of the rigid capillary tube 24 and allows the flushing solution to by-pass this capillary and thereby rapidly fill tubes 14 and 12 to make sure that there is no air present in any of the catheter system.

The present invention provides a valve of extremely simple molded construction having a normally closed position which can be operated very simply by an operator squeezing lever arm 36 towards valve portion 22. This permits one hand operation of the device with complete assurance that the valve will fast flush and the system will be completely sealed when the lever arm is permitted to return to its non-deforming position.

**Claims**

1. A flush valve for use with a catheter, which comprises a relatively rigid, elongated central portion (24), a main body (10) including a flexible outer portion (19) surrounding said central portion (24), said flexible outer portion (19) having a sealing surface (26) which engages said central portion around its whole circumference in liquid sealing relationship, an inlet passageway (16) in said body (10) adjacent one axial end of said central portion (24), an outlet passageway (12) in said body (10) adjacent the other axial end of said central portion (24), and an integral pair of lever arms (19, 21; 36) molded as part of said body (10) and extending radially of an axis parallel to that of the elongated central portion (24), said lever arms being adapted to transmit a shape-deforming force to said flexible outer portion (19) to lift a portion (26) of said sealing surface out of contact with the rigid central portion (24) to permit passage of liquid from said inlet passageway (16) to said outlet passageway (12), said central portion (24) including an axially extending capillary continuously communicating said inlet passageway (16) and said outlet passageway (12).

2. A valve according to claim 1, wherein the rigid central portion (24) is cylindrical and the flexible outer portion (19) contains a circular sealing ring (26) and wherein the deformation force converts the sealing surface from a circle to an oblong to lift a portion of the sealing surface (26) from the central cylindrical portion (24).

3. A valve according to claim 1 or 2, in which the main body (10) includes two parallel tubular portions (19, 21), one (19) of said portions containing the axially extending central portion (24) and the other tubular portion (21) containing a separate passage (22) for connection at one end to a pressure-sensing device and communicating at the other end with the outlet passageway (12), the portion of the main body comprising said two tubular portions (19, 21) constituting one lever arm, the other lever arm (36) being solid and extending at an angle with respect to said main body portion containing said parallel tubular portions (19, 21).

**Revendications**

1. Vanne de rinçage utilisable avec un cathéter, qui comprend une partie centrale (24) allongée et relativement rigide, un corps principal (10) comportant une partie extérieure flexible (19) entourant ladite partie centrale (24), ladite partie extérieure flexible (19) comportant une surface d'étanchéité (26) qui vient s'appliquer contre ladite partie centrale autour de toute sa circonférence dans une relation d'étanchéité au liquide, un passage d'entrée (16) ménagé dans le corps (10) dans une zone adjacente à une extrémité axiale de ladite partie centrale (24), un passage de sortie (12) ménagé dans le corps (10) dans une zone adjacente à l'autre extrémité axiale de ladite partie centrale (24), et une paire de bras de levier (19, 21, 36) solidaires et moulés sous forme d'une partie dudit corps (10) en s'étendant radialement par rapport à un axe parallèle à celui de la partie centrale allongée (24), lesdits bras de levier étant agencés pour transmettre une force de déformation de profil à ladite partie extérieure flexible (19) pour soulever une partie (26) de ladite surface d'étanchéité afin de l'écarter de la partie centrale rigide (24) pour permettre l'écoulement d'un liquide dudit passage d'entrée (16) vers ledit passage de sortie (12), ladite partie centrale (24) comportant un capillaire s'étendant axialement et faisant communiquer de façon continue ledit passage d'entrée (16) avec ledit passage de sortie (12).

2. Vanne selon la revendication 1, caractérisée en ce que la partie centrale rigide (24) est cylindrique, en ce que la partie extérieure flexible (19) comporte une bague circulaire d'étanchéité (26) et en ce que la force de déformation fait passer la surface d'étanchéité d'une forme circulaire à une forme oblongue pour soulever une partie de la surface d'étanchéité (26) à partir de la partie cylindrique centrale (24).

3. Vanne selon l'une des revendications 1 ou 2, caractérisée en ce le corps principal (10) comprend deux parties tubulaires parallèles (19, 21), une (19) desdites parties contenant la partie centrale (24) s'étendant axialement tandis que l'autre partie tubulaire (21) contient un passage séparé (22) pour assurer à une extrémité une liaison avec un dispositif de détection de pression et pour communiquer par l'autre extrémité avec le passage de sortie (12), la partie du corps principal comprenant lesdites deux parties tubulaires (19, 21) constituant un bras de levier, l'autre bras de levier (36) étant massif et s'étendant suivant un certain angle par rapport à ladite partie de corps principal contenant lesdites parties tubulaires parallèles (19, 21).

**Patentansprüche**

1. Spülventil für einen Katheter, mit einem relativ starren, langgestreckten zentralen Teil (24), einem Ventilkörper (10), der einen den zentralen Teil (24) umgebenden, flexiblen äußeren Teil (19) besitzt, der eine Dichtfläche (26) aufweist, die an dem ganzen Umfang des zentralen Teils flüssigkeitsdicht angreift, wobei in dem Ventilkörper (10) in Bereich des einen axialen Endes des zentralen Teils (24) ein Eintrittskanal (16) und im Bereich des anderen axialen Endes des zentralen Teils (24) ein Austrittskanal (12) ausgebildet ist und an den Ventilkörper (10) ein Paar von miteinander einstückigen Hebelarmen (19, 21; 36) angeformt ist, die sich radial in Bezug zu einer Achse erstrecken, die zu der Achse des langgestreckten zentralen Teils (24) parallel ist, die genannten Hebelarme geeignet sind, auf den flexiblen äußeren Teil (19) eine Verformungskraft auszuüben, um einen Teil (26) der Dichtfläche von dem starren zentralen Teil (24) abzuheben, so daß Flüssigkeit aus dem Eintrittskanal (16) in den Austrittskanal (12) gelangen kann, und der zentrale Teil (24) eine axiale Kapillare besitzt, die ständig mit dem Eintrittskanal (16) und dem Austrittskanal (12) in Verbindung steht.

2. Ventil nach Anspruch 1, dadurch gekennzeichnet, daß der starre zentrale Teil (24) zylindrisch ist, der flexible äußere Teil (19) einen kreisförmigen Dichtring (26) enthält und die Verformungskraft die Dichtfläche aus einem Kreis in eine längliche Form verformt, um einen Teil der Dichtfläche (26) von dem zylindrischen zentralen Teil (24) abzuheben.

3. Ventil nach Anspruch 1 oder 2, dadurch gekennzeichnet, daß der Ventilkörper (10) zwei parallele rohrförmige Teile (19, 21) besitzt, von denen der eine (19) den axialen zentralen Teil (24) und der andere (21) einen getrennten Kanal (22) enthält, der an seinem einen Ende mit einer Druckmeßeinrichtung und an seinem anderen Ende mit dem Austrittskanal (12) in Verbindung steht, und daß der diese beiden rohrförmigen Teile (19, 21) umfassende Teile des Ventilkörpers einen der Hebelarme bildet, während der andere Hebelarm (36) voll ist und sich im Winkel zu dem die parallelen rohrförmigen Teile (19, 21) enthaltenden Teil des Ventilkörpers erstreckt.

Fig. 1

Fig. 2

Fig. 3